(19) Europäisches Patentamt
European Patent Office
Office européen des brevets

(11) Veröffentlichungsnummer: **0 320 785**
**A2**

(12) # EUROPÄISCHE PATENTANMELDUNG

(21) Anmeldenummer: **88120490.3**

(22) Anmeldetag: **08.12.88**

(51) Int. Cl.⁴: **C07K 7/10 , G01N 33/86 , A61K 37/02**

Patentansprüche für folgende Vertragsstaaten: ES + GR.

(30) Priorität: **16.12.87 DE 3742633**

(43) Veröffentlichungstag der Anmeldung: **21.06.89 Patentblatt 89/25**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Breipohl, Gerhard, Dr.**
**Geisenheimer Strasse 95**
**D-6000 Frankfurt am Main 71(DE)**
Erfinder: **Henke, Stephan, Dr.**
**Sophienruhe 4**
**D-6232 Bad Soden am Taunus(DE)**
Erfinder: **Knolle, Jochen, Dr.**
**Höchster Strasse 21**
**D-6239 Kriftel(DE)**
Erfinder: **Sandow, Jürgen Kurt, Dr.**
**Am Haideplacken 22**
**D-6240 Königstein/Taunus(DE)**

(54) **Peptide mit beeinflussender Wirkung auf die Hypophyse von Säugern.**

(57) Die Erfindung betrifft neue Analoga (und Derivate) des GRF (Growth Hormone Releasing Factor), Verfahren zu deren Herstellung und deren Verwendung als Heilmittel und Diagnostikum.

EP 0 320 785 A2

## Peptide mit beeinflussender Wirkung auf die Hypophyse von Säugern

Growth Hormone Releasing Factor (GRF) wird im Hypothalamus gebildet und stimuliert die Sekretion des Wachstumshormons in der Hypophyse. Peptide des Typs GRF(1-29)-$NH_2$ sind z. B. im US-Patent 4.626.523 beschrieben.

Die vorliegende Erfindung betrifft Peptide der allgemeinen Formel I

1 2 3 4 5 6 7 8 9 10 11
X - A - B - Asp - Ala - Ile - D - E - F - G - Tyr - Arg -
12 13 14 15 16 17 18 19 20 21 22 23
I - K - Leu - L - Gln - Leu - M - Ala - Arg - I - P - P -
24 25 26 27 28 29
Gln - Q - Ile - R - T - U - Y

in welcher

X Wasserstoff; ($C_1$-$C_8$-Alkyl; ($C_1$-$C_8$)-Alkylcarbonyl; Carboxy-($C_1$-$C_8$)-alkyl; oder einen Rest der Formel II,

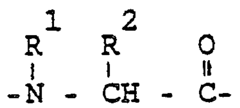

(II)

worin

$R^1$ und $R^2$ zusammen mit den diese Reste tragenden Atomen ein heterocyclisches mono-, bi- oder tricyclisches Ringsystem mit 3-15 C-Atomen bilden, das gegebenenfalls am N-Atom durch ($C_1$-$C_5$)-Alkyl substituiert sein kann, oder fehlt, falls A = 4-Hydroxyphenyl-($C_1$-$C_4$)-alkanoyl, 4-Hydroxyphenoxyacetyl, 4-Halo-phenyl-($C_1$-$C_4$)-alkanoyl, 4-Halo-phenoxyacetyl oder 4-Halo-benzyloxycarbonyl;

A Tyr, (4-Halo)Phe, 3-Carboxy-1,2,3,4-tetrahydroisochinolin, 3-Carboxy-6-hydroxy-1,2,3,4-tetrahydroisochinolin, 3-Carboxy-7-hydroxy-1,2,3,4-tetrahydroisochinolin, S-Carboxymethylcystein, Glu, Aad, wobei die Aminosäure jeweils als L- oder D-Enantiomer vorliegen kann; 4-Hydroxyphenyl-($C_1$-$C_4$)-alkanoyl; 4-Hydroxyphenoxyacetyl; 4-Halo-phenyl-($C_1$-$C_4$)-alkanoyl; 4-Halo-phenoxyacetyl oder 4-Halo-benzyloxycarbonyl;

B D-Ala, D-Leu, D-Ser, D-Thr, D-Phe oder D-3-(2-Thienyl)alanin;

D Phe, (4-Halo)Phe, (4-($C_1$-$C_4$)-Alkoxy)Phe, Trp oder 3-(2-Thienyl)-alanin;

E Thr, Ser oder Gly;

F Asn oder D-Asn;

G Ser, Thr, Ala, Leu, Ile oder Phe;

I Arg, hArg, Lys oder Orn;

K Val, Ile oder Cyclohexyl-alanin;

L Gly, Asp, Glu, Phe, Tyr, Trp oder Gln;

M Ser, Ala, Thr, Leu, Ile, Phe oder Tyr;

P Leu, Phe oder 3-(2-Thienyl)-alanin;

Q Glu, Asp, D-Glu oder D-Asp;

R Nle, Leu, Ile oder O-Methyl-homo-serin;

T eine direkte Bindung, Ser, Gly, Ala, Thr, Ile, Leu, Phe, 3-(2-Thienyl)-alanin oder Gln;

U eine direkte Bindung, Arg oder hArg und

Y -$NH_2$, -NH-$(CH_2)_n$-OH mit n = 2 - 8 oder

falls U oder T und U für eine direkte Bindung stehen -NH-$(CH_2)_n$-$NH_2$, -NH-$(CH_2)_n$-NH-C(=NH)$NH_2$ mit n = 4 - 10, $\beta$-Ala-$NH_2$ oder $\gamma$-Abu-$NH_2$,

mit der Maßgabe, daß im Falle von Y = $NH_2$ X für einen Rest der Formel II stehen muß, mit Ausnahme der folgenden Peptide der Formel I

Ac-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,
H-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,

bedeuten, sowie deren physiologisch verträgliche Salze.

Bevorzugt sind Verbindungen der Formel I, worin

X Wasserstoff; ($C_1$-$C_8$)-Alkyl; ($C_1$-$C_8$)-Alkylcarbonyl; Carboxy-($C_1$-$C_8$)-alkyl oder einen Rest der Formel II;
A 3-Carboxy-1,2,3,4-tetrahydroisochinolin; 3-Carboxy-6-hydroxy-1,2,3,4-tetrahydroisochinolin, 3-Carboxy-7-hydroxy-1,2,3,4-tetrahydroisochinolin, 4-Hydroxyphenyl-($C_1$-$C_4$)-alkanoyl oder 4-Hydroxyphenoxyacetyl;
B D-Ala oder D-Ser;
D Phe oder (4-Halo)Phe;
E Thr oder Ser;
F Asn;
G Ser, Thr oder Ala;
I Arg oder hArg;
K Val oder Ile;
L Gly, Asp, Glu oder Gln;
M Ser, Ala, Thr, Leu oder Ile;
P Leu;
Q Glu oder Asp;
R Nle oder Leu;
T eine direkte Bindung, Ser, Ala, Thr oder Gln;
U eine direkte Bindung, Arg oder hArg und
Y -$NH_2$, oder
falls U oder T und U für seine direkte Bindung stehen
-NH-$(CH_2)_n$-$NH_2$, -NH-$(CH_2)_n$-NH-C(=NH)$NH_2$ mit n = 4 - 10, mit der Maßgabe, daß im Falle von Y = $NH_2$ X für einen Rest der Formel II stehen muß, mit Ausnahme der folgenden Peptide der Formel I
Ac-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,
H-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,

bedeuten, sowie deren physiologisch verträgliche Salze.

Als Rest eines heterocyclischen Ringsystems der Formel II kommen insbesondere Reste von Heterocyclen aus der folgenden Gruppe in Betracht:

Pyrrolidin (A); Piperidin (B); Tetrahydroisochinolin (C); Decahydroisochinolin (D); Octahydroindol (E); Octahydrocyclopenta[b]pyrrol (F); 2-Aza-bicyclo[2.2.2]octan (G); 2-Azabicyclo [2.2.1]heptan (H); 2-Azaspiro-[4.5]decan (I); 2-Azaspiro[4.4]nonan (J); Spiro[(bicyclo[2.2.1]heptan)-2,3-pyrrolidin] (K); Spiro[(bicyclo[2.2.2]-octan)2,3-pyrrolidin] (L); 2-Azatricyclo [4.3.0.1 $^{6,9}$] decan (M); Decahydrocyclohepta[b]pyrrol (N); Octahydroisoindol (O); Octahydrocyclopenta[c]pyrrol (P); 2,3,3a,4,5,7a-Hexahydroindol (Q); Tetrahydrothiazol (R); 2-Azabicyclo [3.1.0]hexan (S); die alle gegebenenfalls substituiert sein können.

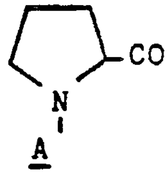
A

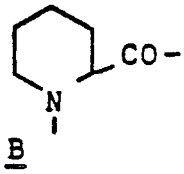
B

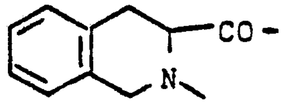
C

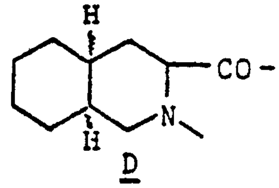
D

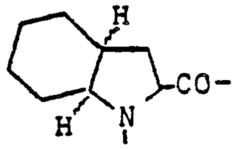
E

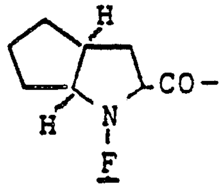
F

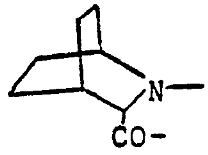
G

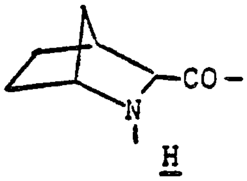
H

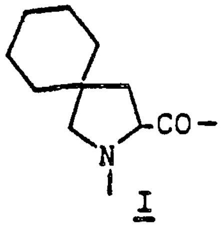
I

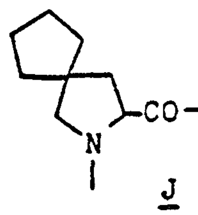
J

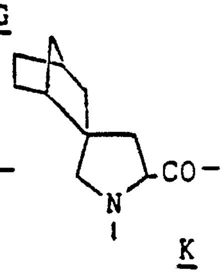
K

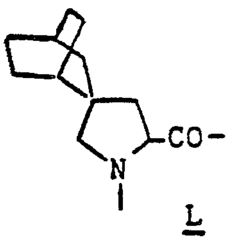
L

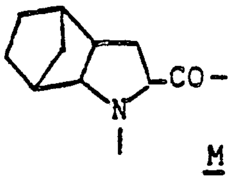
M

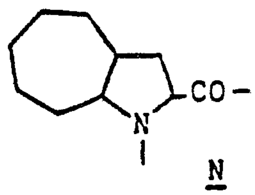
N

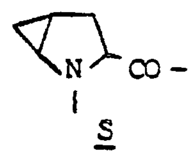
S

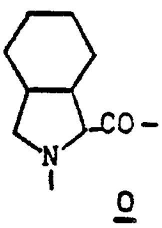
O

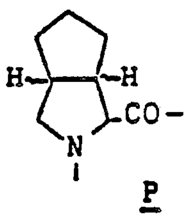
P

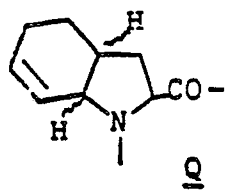
Q

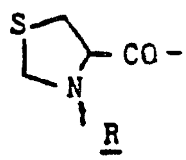
R

Die oben genannten Resten zugrundeliegenden Heterocyclen sind beispielsweise bekannt aus

US-PS 4 344 949 US-PS 4 374 847, US-PS 4 350 704,
EP-A 50 800, EP-A 31 741, EP-A 51 020, EP-A 49 658,
EP-A 49 605, EP-A 29 488, EP-A 46 953, EP-A 52 870,
DE-A 32 26 768, DE-A 31 51 690, DE-A 32 10 496,
DE-A 32 11 397, DE-A 32 11 676, DE-A 32 27 055,
DE-A 32 42 151, DE-A 32 46 503 und DE-A 32 46 757.

Falls im Einzelfall nicht anders angegeben, kann Alkyl geradkettig oder verzweigt sein. Entsprechendes gilt für davon abgeleitete Reste wie Alkoxy oder Alkanoyl.

Halo steht für Fluor, Chlor, Brom oder Jod, vorzugsweise für Chlor.

Als Salze kommen insbesondere Alkali- oder Erdalkalisalze, Salze mit physiologisch verträglichen Aminen und Salze mit anorganischen oder organischen Säuren wie z.B. HCl, HBr, $H_2SO_4$, $H_3PO_4$, Maleinsäure, Furmarsäure, Citronensäure, Weinsäure, Essigsäure in Frage.

Falls nicht anders angegeben, steht die Abkürzung eines Aminosäurerestes ohne eine Stereodeskriptor für den Rest in der L-Form (vgl. Schröder, Lübke, The Peptides, Band I, New York 1965, Seiten XXII-XXIII; Houben-Weyl, Methoden der Organischen Chemie, Band XV/1 und 2, Stuttgart 1974, Beilage Seiten 1 - 2).

Die Erfindung betrifft weiterhin ein Verfahren zur Herstellung von Peptiden der Formel I, das dadurch

gekennzeichnet ist, daß man

a) ein Fragment mit C-terminaler freier Carboxylgruppe oder dessen aktiviertes Derivat mit einem entsprechenden Fragment mit N-terminaler freier Aminogruppe umsetzt

oder

b) das Peptid stufenweise aufbaut,

in der nach (a) oder (b) erhaltenen Verbindung gegebenenfalls eine oder mehrere zum Schutz anderer Funktionen temporär eingeführte Schutzgruppen abspaltet und die so erhaltenen Verbindungen der Formel I gegebenenfalls in ihre physiologisch verträglichen Salze überführt.

Die Peptide der vorliegenden Erfindung werden nach allgemein bekannten Methoden der Peptidchemie, siehe z.B. Houben-Weyl, Methoden der organischen Chemie, Band 15/2, bevorzugt mittels Festphasensynthese wie z.B. von B. Merrifield, J. Am. Chem. Soc. 85, 2149 (1963) oder R. C. Sheppard, Int. J. Peptide Protein Res. 21, 118 (1983) beschrieben, oder nach äquivalenten bekannten Methoden hergestellt. Als α-Aminoschutzgruppe werden Urethanschutzgruppen wie z.B. die tert.-Butyloxycarbonyl (Boc)- oder Fluorenylmethyloxycarbonyl(Fmoc-Schutzgruppe verwendet. Falls zur Verhinderung von Nebenreaktionen oder für die Synthese spezieller Peptide erforderlich, sind die funktionellen Gruppen in der Seitenkette von Aminosäuren zusätzlich geschützt.

Die Festphasensynthese beginnt am C-terminalen Ende des Peptides mit der Kupplung einer geschützten Aminosäure an ein entsprechendes Harz. Derartige Ausgangsmaterialien können durch Verknüpfung einer geschützten Aminosäure mit einem mit einer Chlormethyl-, Hydroxymethyl-, Benzhydrylamino(BHA)-, Methylbenzhydrylamino(MBHA)-Gruppe modifiziertem Polystyrol- oder Polyacrylamid-Harz über eine Ester- bzw. Amidbindung erhalten werden.

Weiterhin können Harze mit speziellen Ankergruppen, wie sie z.B. in EP-A 264 802, in EP-A 287 882 oder bei Schally et al.

(Abstracts of the 10th American Peptide Symposium) beschrieben sind, verwendet werden. BHA- und MBHA- und die in EP-A 287 882 beschriebenen Harze sowie die in der deutschen Patentanmeldung P 37 43 6201 vorgeschlagenen Harze werden für gewöhnlich verwendet wenn das synthetisierte Peptid am C-Terminus eine freie Amidgruppierung enthalten soll.

Nach Abspaltung der Aminoschutzgruppe der an das Harz gekuppelten Aminosäure mit einem geeigneten Reagenz, wie z.B. Trifluoressigsäure in Methylenchlorid im Falle der Boc-Schutzgruppe oder einer 20 %igen Lösung von Piperidin in Dimethylformamid im Falle der Fmoc-Schutzgruppe, werden die nachfolgenden geschützten Aminosäuren nacheinander in der gewünschten Reihenfolge aufgekuppelt.

Die intermediär entstehenden N-terminal geschützten Peptidharze werden vor der Verknüpfung mit dem nachfolgenden Aminosäurederivat durch die vorbeschriebenen Reagenzien entblockiert.

Als Kupplungsreagenz können alle möglichen in der Peptidsynthese verwendeten Aktivierungs-Reagenzien, siehe z.B. Houben-Weyl, Methoden der organischen Chemie, Band 15/2, verwendet werden, insbesondere aber Carbodiimide wie z.B. N,N′-Dicyclohexylcarbodiimid,

N,N′-Diisopropylcarbodiimid oder N-ethyl-N′-(3-dimethylaminopropyl)carbodiimid. Die Kupplung kann dabei direkt durch Addition des Aminosäurederivats mit dem Aktivierungsreagenz und gegebenenfalls einem die Racemisierung unterdrückenden Zusatz wie z.B. 1-Hydroxybenzotriazol (HOBt) (W. König, R. Geiger, Chem. Ber. 103, 788 (1970)) oder 3-Hydroxy-4-oxo-3,4-dihydrobenzotriazin (HOObt) (W. König, R. Geiger, Chem. Ber. 103, 2034 (1970)) zum Harz durchgeführt werden oder aber die Voraktivierung des Aminosäurederivats als symmetrisches Anhydrid oder HOBt- bzw. HOObt-Ester kann separat erfolgen und die Lösung der aktivierten Spezies in einem geeigneten Lösungsmittel zum kupplungsfähigen Peptidharz gegeben werden.

Ebenso können isolierte HOObt-Ester, wie sie z.B. in EP-A 247 573 beschrieben sind, zur Synthese eingesetzt werden.

Die Kupplung bzw. Aktivierung der Aminosäurederivate mit einem der oben genannten Aktivierungsreagenzien kann in Dimethylformamid, N-Methylpyrrolidon, Methylenchlorid oder einer Mischung dieser Lösungsmittel durchgeführt werden. Das aktivierte Aminosäurederivat wird üblicherweise in einem 1,5- bis 4-fachen Überschuß eingesetzt. In Fällen, in denen eine unvollständige Kupplung eintritt, wird die Kupplungsreaktion wiederholt ohne vorher die für die Kupplung der nächstfolgenden Aminosäure nötige Entblockierung der α-Aminogruppe des Peptidharzes durchzuführen.

Der erfolgreiche Ablauf der Kupplungsreaktion kann mittels der Ninhydrin-Reaktion, wie z.B. von E. Kaiser et al., Anal. Biochem. 34 595 (1970) beschrieben, überprüft werden.

Die Synthese kann auch automatisiert z.B. mit einem Peptid-Synthesizer Modell 430A der Fa. Applied Biosystems durchgeführt werden, wobei entweder die vom Gerätehersteller vorgesehenen Syntheseprogramme oder aber auch vom Benutzer selbst erstellte verwendet werden können. Letztere werden insbesondere bei der Verwendung von mit der Fmoc-Gruppe geschützten Aminosäurederivaten eingesetzt.

Nach der Synthese der Peptide in der vorgehend beschriebenen Weise kann das Peptid vom Harz mit Reagenzien wie z.B. flüssigem Fluorwasserstoff, Trifluoressigsäure oder einem Gemisch aus Trifluormethansulfonsäure und Trifluoressigsäure abgespalten werden. Diese Reagenzien spalten nicht nur das Peptid vom Harz sondern gewöhnlich auch die weiteren Seitenkettenschutzgruppen der Aminosäurederivate. Auf diese Weise erhält man je nach verwendetem Harz das Peptid in Form des Amides. Bei der Abspaltung mit den oben genannten Säuren oder Säuregemischen werden als Kationenfänger üblicherweise Substanzen wie Phenol, Kresol, Thiokresol, Thioanisol, Dimethylsulfid, Ethylmethylsulfid oder eine Mischung von zwei oder mehreren dieser Hilfsmittel zugesetzt. Die Trifluoressigsäure kann dabei auch durch geeignete Lösungsmittel wie z.B. Methylenchlorid verdünnt angewendet werden.

Die Peptide der vorliegenden Erfindung werden insbesondere mittels Festphasensynthese unter Verwendung der Fmoc-Schutzgruppe zum temporären Schutz der $\alpha$-Aminogruppe hergestellt.
Die Aktivierung der Aminosäurederivate als HOBt- oder HOObt-Ester erfolgt direkt in den vom Gerätehersteller gelieferten Aminosäurecartridges durch Zugabe einer Lösung von Diisopropylcarbodiimid in Dimethylformamid zu der vorher eingewogenen Mischung aus Aminosäurederivat und HOBt bzw. HOObt. Bei der Verwendung von zuvor hergestellten HOObt-Estern werden diese in die Cartridges eingewogen und mit Dimethylformamid (DMF) oder N-Methylpyrrolidon (NMP) gelöst und in das Reaktionsgefäß transferiert.

Die Abspaltung der Fmoc-Schutzgruppe erfolgt mit einer 20 %igen Lösung von Piperidin in DMF im Reaktionsgefäß. Der verwendete Überschuß an reaktivem Aminosäurederivat beträgt 1,5 bis 2,5 Äquivalente. Falls die Kupplung nicht vollständig ist, wird sie wiederholt ohne vorher die Schutzgruppe abzuspalten.

Ein typischer Synthesezyklus ist nachfolgend aufgelistet:

1) Abspaltung der Fmoc-Gruppe mit 20 % Piperidin in DMF (zweimal 8 ml je 10 Minuten)

2) Waschen mit DMF bzw. NMP (6mal mit je 8 ml DMF bzw. NMP)

3) Kupplung des in der Cartridge als HOBt- oder HOObt-Ester voraktivierten Aminosäurederivats bzw. des in DMF oder NMP gelösten HOObt-Esters (25-45 Minuten), evtl. Wiederholung bei unvollständiger Kupplung

4) Waschen mit DMF oder NMP (6mal mit je 8 ml DMF oder NMP)

Nach beendeter Synthese wird vom Peptidharz zunächst die Fmoc-Schutzgruppe durch Behandlung mit 20 % Piperidin in DMF abgespalten und das gut mit DMF gewaschene Harz durch mehrfache Behandlung mit Isopropanol und Methyl-tert.-butylether geschrumpft. Nach Trocknung im Hochvakuum wird das Peptid durch Rühren mit einer Mischung aus Trifluoressigsäure/$CH_2Cl_2$/Phenol (70:30:5), 4 h bei Raumtemperatur vom Harz abgespalten. Durch Nachbehandlung mit Trifluoressigsäure/Phenol/Dimethylsulfid (90:5:5) werden evtl. verbliebene Schutzgruppen abgespalten. Anschließend wird vom Harz abfiltriert, mit Trifluoressigsäure/Phenol/Dimethylsulfid nachbehandelt und das Filtrat im Vakuum eingeengt.

Durch mehrfaches Verrühren mit Essigester werden die Kationenfänger entfernt. Nach Trocknung im Hochvakuum wird das Rohpeptid mit 1 N Essigsäure an ®Sephadex G25 superfine von evtl. noch vorhandenem Kationenfänger und Trifluoressigsäurespuren abgetrennt. Die peptidhaltigen Fraktionen werden gefriergetrocknet und dann weiter aufgearbeitet. Die weitere Reinigung erfolgt gegebenenfalls durch präparative HPLC-Trennung.

Die erfindungsgemäßen Peptide der Formel I bewirken die Ausschüttung des Wachstumshormons in der Hypophyse. Diese Peptide können deshalb als Diagnostikum dafür dienen, ob das Wachstumshormon freigesetzt wird. Andererseits dienen die erfindungsgemäßen Peptide als Heilmittel bei Wachstumsstörungen, also zur Steigerung des Wachstums bei Kindern und Jugendlichen. Darüber hinaus zeigen die Peptide der Formel I eine Reihe von weiteren Wirkungen, die ihren Einsatz bei der Behandlung von Krankheiten Erwachsener erlauben:Sie fördern die Wund- und Knochenbruchheilung und bewirken einen Aufbau von Eiweiß bei konsumierenden Prozessen mit starker Eiweißeinschmelzung.

Die Verbindungen der Formel I können auch in der Veterinärmedizin bzw. zur Wachstumsbeschleunigung in der Tierzucht und zur Steigerung der Milchleistung eingesetzt werden.

Die Verbindungen der Formel I zeigen im Organismus eine GRF-Wirkung (Freisetzung des Wachstumshormons), die in quantitativer Beziehung der des natürlichen GRF bzw. der entsprechenden Analoga weitgehend entspricht. Die in der Human- und/oder Veterinärmedizin anzuwendende Dosis entspricht somit auch der jeweils für GRF bzw. dessen Analoga unter Berücksichtigung der spezifischen biologischen Wirkstärke dieser Verbindungen anzuwendenden Dosis.

Die Methoden zur Bestimmung der biologischen Aktivität der GRF-Peptide sind z. B. in L. Frohman, J. Jansson, Endocrine Reviews 7 (1986) 223 angegeben. Der in-vitro-Test erfolgte durch Inkubation von Hypophysen männlicher Ratten mit einer Lösung des jeweiligen Peptids in verschiedenen Konzentrationen. Der Growth-Hormon-Gehalt der Hypophysen und des Mediums wurde durch Radioimmunoassay nach der

bei D. S. Schalch, S. Reichlin, Endocrinology 79 (1966) 275 beschriebenen Methode bestimmt. Der in-vivo-Test wurde an Urethan-anästhesierten männlichen Ratten durch i. v. Injektion von Peptidlösungen in verschiedenen Konzentrationen durchgeführt. Die Plasma-Konzentration an Growth-Hormon wurde durch Radioimmunoassay wie oben beschrieben bestimmt.

Die erfindungsgemäßen Verbindungen können parenteral oder auf die Schleimhaut - bevorzugt intranasal - in entsprechender pharmazeutischer Zubereitung verabreicht werden. Zur Applikation auf die Schleimhäute werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Pastillen, wäßrige, alkoholische oder ölige Suspensionen oder wäßrige, alkoholische oder ölige Lösungen. Als inerte Träger können z. B. Gummi arabicum, Magnesia, Magnesiumcarbonat, Milchzucker, Glucose und Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren, oder weitere Hilfsstoffe in Lösung, Suspension oder Emulsion gebracht. Als Lösungsmittel für die neuen aktiven Verbindungen und die entsprechenden physiologisch verträglichen Salze kommen z. B. in Frage: Wasser, physiologische Kochsalzlösungen oder Alkohole, z. B. Ethanol, Propandiol, oder Glycerin, daneben auch Zuckerlösungen wie Glucose- oder Mannitlösungen, oder auch eine Mischung aus den verschiedenen genannten Lösungsmitteln. Besonders wirkungsvoll ist eine pulsatile Applikation mit Pausen von etwa 3 Stunden (Pumpe). Auch slow release-Zubereitungen, die als Rods oder Mikrokapseln injiziert werden können, sind möglich. Träger für biodegradierbare slow release-Zubereitungen sind z. B. Polymerisate und Co-Polymerisate aus Glykolsäure, Milchsäure und Poly-3-hydroxybuttersäure.

Die für die Aminosäuren verwendeten Abkürzungen entsprechen dem in der Peptidchemie üblichen drei Buchstaben-Code wie er z.B. in Europ. J. Biochem. 138, 9 (1984) beschrieben ist. Weitere Abkürzungen sind nachfolgend aufgelistet;

| | |
|---|---|
| Aad | α-Aminoadipinsäure |
| γAbu | γ-Aminobuttersäure |
| Ac | Acetyl |
| Aoc | cis, endo-2-Azabicyclo[3.3.0]octan-3-S-carbonyl |
| BHA | Benzhydrylamino |
| Boc | tert.-Butyloxycarbonyl |
| DMF | Dimethylformamid |
| Fmoc | 9-Fluorenylmethyloxycarbonyl |
| hArg | Homoarginin |
| HOBt | 1-Hydroxybenzotriazol |
| HOObt | 3-Hydroxy-4-oxo-3,4-dihydrobenzotriazin |
| MBHA | Methylbenzhydrylamino |
| Mtr | 4-Methoxy-2,3,6-trimethylphenylsulfonyl |
| Nle | Norleucin |
| NMP | N-Methylpyrrolidon |
| TFA | Trifluoressigsäure |
| Tic | 3-Carboxy-1,2,3,4-tetrahydroisochinolin |

Die nachfolgenden Beispiele sollen die Herstellung der erfindungsgemäßen Peptide verdeutlichen ohne jedoch darauf beschränkt zu sein:

**Beispiel 1:**

Synthese von [D-Ala$^2$,Nle$^{27}$]-GRF(1-28)-6-aminohexylamid mit der Sequenz

1 2 3 4 5 6 7 8 9 10 11 12 13
H-Tyr-D-Ala-Asp-Ala-Ile-Phe-Thr-Asn-Ser-Tyr-Arg-Lys-Val-

14 15 16 17 18 19 20 21 22 23 24 25 26 27
Leu-Gly-Gln-Leu-Ser-Ala-Arg-Lys-Leu-Leu-Gln-Asp-Ile-Nle-

28
Ser-NH-$(CH_2)_6$-$NH_2$

Die Peptidsynthese erfolgte an 1 g eines Aminomethylharzes, das mit einer in der EP-A 264 802 beschriebenen Ankergruppe vom Typ

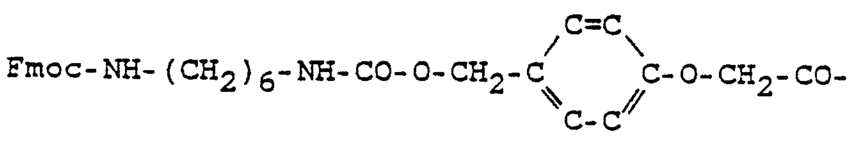

modifiziert war, unter Verwendung von Fmoc-Aminosäure-OObt Estern mit einem automatischen Peptidsynthesizer (Modell 430A der Fa. Applied Biosystems) und selbst modifizierten Syntheseprogrammen. Dazu wurden jeweils 1 mMol des entsprechenden Aminosäurederivates in die vom Hersteller gelieferten Cartridges eingewogen, Fmoc-Arg(Mtr)-OH, Fmoc-Asn-OH und Fmoc-Gln-OH wurden zusammen mit 0,95 mMol HOObt in die Cartridges eingewogen. Die Voraktivierung dieser Aminosäuren erfolgte direkt in den Cartridges durch Lösen in 4 ml DMF und Zugabe von 2 ml einer 0,55 M Lösung von Diisopropylcarbodiimid in DMF. Die HOObt-Ester der anderen Aminosäuren wurden in 6 ml NMP gelöst und dann ebenso wie die in situ voraktivierten Aminosäuren Arginin, Asparagin und Glutamin an das vorher mit 20 % Piperidin in DMF entblockierte Harz gekuppelt, wobei die in situ aktivierten Aminosäuren doppelt gekuppelt wurden. Nach beendeter Synthese wurde das Peptid-6-aminohexylamid unter gleichzeitiger Entfernung der Seitenkettenschutzgruppen mit Trifluoressigsäure, die Thioanisol und m-Kresol als Kationenfänger enthielt, vom Harz abgespalten. Der nach Abziehen der Trifluoressigsäure erhaltene Rückstand wurden mehrfach mit Essigester digeriert und zentrifugiert. Das verbliebene Rohpeptid wurde an ®Sephadex G25 mit 1N Essigsäure chromatographiert. Die das reine Peptid enthaltenden Fraktionen wurden vereinigt und gefriergetrocknet.

**Beispiel 2**

Aoc-[$Ser^7$,$Ala^9$,$Ile^{13}$,$Gln^{15}$,$Leu^{18}$,$Leu^{27}$,$Gln^{28}$]-hGRF(1-29)amid mit der Sequenz

1 2 3 4 5 6 7 8 9 10 11 12 13
H-Aoc-Tyr-Ala-Asp-Ala-Ile-Phe-Ser-Asn-Ala-Tyr-Arg-Lys-Ile-

14 15 16 17 18 19 20 21 22 23 24 25 26 27
Leu-Gln-Gln-Leu-Leu-Ala-Arg-Lys-Leu-Leu-Gln-Asp-Ile-Leu-

28 29
Gln-Arg-$NH_2$

wurde an 1 g MBHA-Harz hergestellt. Dazu wurde als erste Aminosäure Fmoc-Arg(Mtr)-OH als in situ voraktiverter HOObt-Ester an das Harz gekuppelt.

Die Peptidsynthese erfolgte dann wie in Beispiel 1 beschrieben unter Verwendung von Fmoc-Aminosäure-OObt Estern mit einem automatischen Peptidsynthesizer (Modell 430A der Fa. Applied Biosystems) und selbstmodifizierten Syntheseprogrammen. Dazu wurden jeweils 1 mMol des entsprechenden Aminosäurederivates in die vom Hersteller gelieferten Cartridges eingewogen. Fmoc-Arg(Mtr)-OH, Fmoc-Asn-OH und Fmoc-Gln-OH wurden zusammen mit 0,95 mMol HOObt in die Cartridges eingewogen. Die

Voraktivierung dieser Aminosäuren erfolgte direkt in den Cartridges durch Lösen in 4 ml DMF und Zugabe von 2 ml einer 0,55 M Lösung von Diisopropylcarbodiimid in DMF. Die HOObt-Ester der anderen Aminosäuren wurden in 6 ml NMP gelöst und dann ebenso wie die in situ voraktiverten Aminosäuren Arginin, Asparagin und Glutamin an das vorher mit 20 % Piperidin in DMF entblockierte Harz gekuppelt. Die in situ aktiverten Aminosäuren wurden doppelt gekuppelt. Nach beendeter Synthese wurden vom Peptidamid zunächst mit Trifluoressigsäure, die Thioanisol und m-Kresol als Kationenfänger enthielt, die Seitenkettenschutzgruppen entfernt und dann das Peptidamid mit Trifluoressigsäure, die 10 % Trifluormethansulfonsäure und die obigen Scavenger enthielt, bei zunächst 0 °C und dann bei Raumtemperatur vom Harz abgespalten. Danach wurde vom Harz in eiskalten, gut gerührten Methyl-tert.Butylether abgesaugt und mit wenig TFA nachgewaschen. Der nach Zentrifugation erhaltene Niederschlag wurde nochmals mit Methyl-tert. Butylether digeriert, zentrifugiert und im Vakuum von Lösungsmittelresten befreit. Anschließend wurde der Niederschlag in Wasser gelöst und sofort mit Anionenaustauscher in die Acetatform überführt. Das Rohpeptid wurde an ®Sephadex G25 mit 1N Essigsäure chromatographiert. Die das reine Peptid enthaltenden Fraktionen wurden vereinigt und gefriergetrocknet.

Analog Beispiel 1 wurden hergestellt

**Beispiel 3**

[D-$Ala^2$,$Ala^9$,$Ile^{13}$,$Gln^{15}$,$Leu^{18}$,$Nle^{27}$]-GRF(1-28)-8-aminooctylamid
mit der Sequenz

```
    1      2    3    4    5    6    7    8    9    10   11   12   13
H-Tyr-D-Ala-Asp-Ala-Ile-Phe-Thr-Asn-Ala-Tyr-Arg-Lys-Ile-

14  15  16  17  18  19  20  21  22  23  24  25  26  27
Leu-Gln-Gln-Leu-Leu-Ala-Arg-Lys-Leu-Leu-Gln-Asp-Ile-Nle-

28
Ser-NH-(CH2)8-NH2
```

Analog Beispiel 2 wurden hergestellt

**Beispiel 4**

Aoc-[D-$Ala^2$,$Ser^7$,$Ala^9$,$Ile^{13}$,$Gln^{15}$,$Leu^{18}$,$Nle^{27}$]-hGRF(1-29)amid
mit der Sequenz

```
        1      2    3    4    5    6    7    8    9    10   11   12
H-Aoc-Tyr-D-Ala-Asp-Ala-Ile-Phe-Ser-Asn-Ala-Tyr-Arg-Lys-

13  14  15  16  17  18  19  20  21  22  23  24  25  26
Ile-Leu-Gln-Gln-Leu-Leu-Ala-Arg-Lys-Leu-Leu-Gln-Asp-Ile-

27  28  29
Nle-Ser-Arg-NH2
```

**Beispiel 5**

[$Tic^1$,D-$Ala^2$,$Ser^7$,$Ala^9$,$Ile^{13}$,$Gln^{15}$,$Leu^{18}$,$Nle^{27}$]-hGRF(1-29)amid
mit der Sequenz

H-Tic-D-Ala-Asp-Ala-Ile-Phe-Ser-Asn-Ala-Tyr-Arg-Lys-Ile-Leu-Gln-Gln-Leu-Leu-Ala-Arg-Lys-Leu-Leu-Gln-Asp-Ile-Nle-Ser-Arg-$NH_2$

(Positionen 1–29)

Analog Beispiel 1 wurden hergestellt

**Beispiel 6**

[D-Ala[2],Gln[15],Nle[27],Gln[28]]-GRF(1-28)-8-aminooctylamid mit der Sequenz

H-Tyr-D-Ala-Asp-Ala-Ile-Phe-Thr-Asn-Ser-Tyr-Arg-Lys-Val-Leu-Gln-Gln-Leu-Ser-Ala-Arg-Lys-Leu-Leu-Gln-Asp-Ile-Nle-Gln-NH-$(CH_2)_8$-$NH_2$

(Positionen 1–28)

**Beispiel 7**

[D-Ala[2],Gln[15],Nle[27],Gln[28]]-GRF(1-28)-6-aminohexylamid mit der Sequenz

H-Tyr-D-Ala-Asp-Ala-Ile-Phe-Thr-Asn-Ser-Tyr-Arg-Lys-Val-Leu-Gln-Gln-Leu-Ser-Ala-Arg-Lys-Leu-Leu-Gln-Asp-Ile-Nle-Gln-NH-$(CH_2)_6$-$NH_2$

(Positionen 1–28)

**Beispiel 8**

Aoc-[D-Ala[2],Gln[15],Nle[27],Gln[28]]-GRF(1-28)-8-aminooctylamid mit der Sequenz

1 2 3 4 5 6 7 8 9 10 11 12
H-Aoc-Tyr-D-Ala-Asp-Ala-Ile-Phe-Thr-Asn-Ser-Tyr-Arg-Lys-

13 14 15 16 17 18 19 20 21 22 23 24 25 26
Val-Leu-Gln-Gln-Leu-Ser-Ala-Arg-Lys-Leu-Leu-Gln-Asp-Ile-

27 28
Nle-Gln-NH-$(CH_2)_8$-$NH_2$

**Beispiel 9**

Aoc-[D-Ala$^2$,Gln$^{15}$,Nle$^{27}$,Gln$^{28}$]-GRF(1-28)-6-aminohexylamid mit der Sequenz

1 2 3 4 5 6 7 8 9 10 11 12
H-Aoc-Tyr-D-Ala-Asp-Ala-Ile-Phe-Thr-Asn-Ser-Tyr-Arg-Lys-

13 14 15 16 17 18 19 20 21 22 23 24 25 26
Val-Leu-Gln-Gln-Leu-Ser-Ala-Arg-Lys-Leu-Leu-Gln-Asp-Ile-

27 28
Nle-Gln-NH-$(CH_2)_6$-$NH_2$

**Beispiel 10**

[D-Ala$^2$,Nle$^{27}$]-GRF(1-27)-8-aminooctylamid mit der Sequenz

1 2 3 4 5 6 7 8 9 10 11 12
H-Tyr-D-Ala-Asp-Ala-Ile-Phe-Thr-Asn-Ser-Tyr-Arg-Lys-

13 14 15 16 17 18 19 20 21 22 23 24 25 26
Val-Leu-Gly-Gln-Leu-Ser-Ala-Arg-Lys-Leu-Leu-Gln-Asp-Ile-

27
Nle-NH-$(CH_2)_8$-$NH_2$

**Beispiel 11**

4-Hydroxyphenoxyacetyl-[D-Ala$^2$,Nle$^{27}$]-GRF(1-28)-6-aminohexylamid mit der Sequenz

1 2 3 4 5 6 7 8 9 10 11 12
4-HPA-Tyr-D-Ala-Asp-Ala-Ile-Phe-Thr-Asn-Ser-Tyr-Arg-Lys-

13 14 15 16 17 18 19 20 21 22 23 24 25 26
Val-Leu-Gly-Gln-Leu-Ser-Ala-Arg-Lys-Leu-Leu-Gln-Asp-Ile-

27 28
Nle-Ser-$NH-(CH_2)_6-NH_2$

**Beispiel 12**

4-Hydroxyphenylpropionyl-[D-$Ala^2$,$Nle^{27}$]-GRF(1-28)-6-aminohexylamid mit der Sequenz

1 2 3 4 5 6 7 8 9 10 11 12
4-HPP-Tyr-D-Ala-Asp-Ala-Ile-Phe-Thr-Asn-Ser-Tyr-Arg-Lys-

13 14 15 16 17 18 19 20 21 22 23 24 25 26
Val-Leu-Gly-Gln-Leu-Ser-Ala-Arg-Lys-Leu-Leu-Gln-Asp-Ile-

27 28
Nle-Ser-$NH-(CH_2)_6-NH_2$

**Beispiel 13**

[D-$Ala^2$.$Nle^{27}$]-GRF(1-28)-4-aminobutylamid mit der Sequenz

1 2 3 4 5 6 7 8 9 10 11 12 13
H-Tyr-D-Ala-Asp-Ala-Ile-Phe-Thr-Asn-Ser-Tyr-Arg-Lys-Val-

14 15 16 17 18 19 20 21 22 23 24 25 26 27
Leu-Gly-Gln-Leu-Ser-Ala-Arg-Lys-Leu-Leu-Gln-Asp-Ile-Nle-

28
Ser-$NH-(CH_2)_4-NH_2$

**Beispiel 14**

Aoc-[D-$Ala^2$,$Nle^{27}$]-GRF(1-28)-6-aminohexylamid mit der Sequenz

1 2 3 4 5 6 7 8 9 10 11 12
H-Aoc-Tyr-D-Ala-Asp-Ala-Ile-Phe-Thr-Asn-Ser-Tyr-Arg-Lys-

13 14 15 16 17 18 19 20 21 22 23 24 25 26
Val-Leu-Gly-Gln-Leu-Ser-Ala-Arg-Lys-Leu-Leu-Gln-Asp-Ile-

27 28
Nle-Ser-$NH-(CH_2)_6-NH_2$

**Beispiel 15**

D-Aoc-[D-Ala$^2$,Nle$^{27}$]-GRF(1-28)-6-aminohexylamid mit der Sequenz

```
        1      2   3    4   5   6   7   8   9   10  11  12
H-D-Aoc-Tyr-D-Ala-Asp-Ala-Ile-Phe-Thr-Asn-Ser-Tyr-Arg-Lys-

13  14  15  16  17  18  19  20  21  22  23  24  25  26
Val-Leu-Gly-Gln-Leu-Ser-Ala-Arg-Lys-Leu-Leu-Gln-Asp-Ile-

27  28
Nle-Ser-NH-(CH2)6-NH2
```

**Beispiel 16**

D-Ala$^2$,Nle$^{27}$]-GRF(1-27)-6-aminohexylamid mit der Sequenz

```
  1      2   3   4   5   6   7   8   9   10  11  12  13
H-Tyr-D-Ala-Asp-Ala-Ile-Phe-Thr-Asn-Ser-Tyr-Arg-Lys-Val-

14  15  16  17  18  19  20  21  22  23  24  25  26  27
Leu-Gly-Gln-Leu-Ser-Ala-Arg-Lys-Leu-Leu-Gln-Asp-Ile-Nle-

NH-(CH2)6-NH2
```

**Beispiel 17**

Aoc-[D-Ala$^2$,Nle$^{27}$]-GRF(1-27)-8-aminooctylamid mit der Sequenz

```
      1      2   3   4   5   6   7   8   9   10  11  12
H-Aoc-Tyr-D-Ala-Asp-Ala-Ile-Phe-Thr-Asn-Ser-Tyr-Arg-Lys-

13  14  15  16  17  18  19  20  21  22  23  24  25  26
Val-Leu-Gly-Gln-Leu-Ser-Ala-Arg-Lys-Leu-Leu-Gln-Asp-Ile-

27
Nle-NH-(CH2)8-NH2
```

**Beispiel 18**

[D-Ala$^2$,Nle$^{27}$]-GRF(1-27)-4-aminobutylamid mit der Sequenz

H-Tyr[1]-D-Ala[2]-Asp[3]-Ala[4]-Ile[5]-Phe[6]-Thr[7]-Asn[8]-Ser[9]-Tyr[10]-Arg[11]-Lys[12]-Val[13]-

Leu[14]-Gly[15]-Gln[16]-Leu[17]-Ser[18]-Ala[19]-Arg[20]-Lys[21]-Leu[22]-Leu[23]-Gln[24]-Asp[25]-Ile[26]-Nle[27]-

$NH-(CH_2)_4-NH_2$

**Beispiel 19**

[D-Ala$^2$]-GRF(1-26)-8-aminooctylamid mit der Sequenz

H-Tyr[1]-D-Ala[2]-Asp[3]-Ala[4]-Ile[5]-Phe[6]-Thr[7]-Asn[8]-Ser[9]-Tyr[10]-Arg[11]-Lys[12]-Val[13]-

Leu[14]-Gly[15]-Gln[16]-Leu[17]-Ser[18]-Ala[19]-Arg[20]-Lys[21]-Leu[22]-Leu[23]-Gln[24]-Asp[25]-Ile[26]-NH-

$(CH_2)_8-NH_2$

**Beispiel 20**

[D-Asp$^3$,D-Asn$^8$]-GRF(1-26)-4-aminobutylamid mit der Sequenz

H-Tyr[1]-Ala[2]-D-Asp[3]-Ala[4]-Ile[5]-Phe[6]-Thr[7]-D-Asn[8]-Ser[9]-Tyr[10]-Arg[11]-Lys[12]-Val[13]-

Leu[14]-Gly[15]-Gln[16]-Leu[17]-Ser[18]-Ala[19]-Arg[20]-Lys[21]-Leu[22]-Leu[23]-Gln[24]-Asp[25]-Ile[26]-NH-

$(CH_2)_4-NH_2$

## Ansprüche

1. Verbindung der Formel I

1 2 3 4 5 6 7 8 9 10 11
X - A - B - Asp - Ala - Ile - D - E - F - G - Tyr - Arg -
12 13 14 15 16 17 18 19 20 21 22 23
I - K - Leu - L - Gln - Leu - M - Ala - Arg - I - P - P -
24 25 26 27 28 29
Gln - Q - Ile - R - T - U - Y

in welcher

X Wasserstoff; ($C_1$-$C_8$)-Alkyl; ($C_1$-$C_8$)-Alkylcarbonyl; Carboxy-($C_1$-$C_8$)-alkyl; oder einen Rest der Formel II,

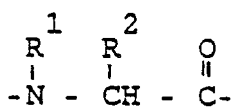

(II)

worin

$R^1$ und $R^2$ zusammen mit den diese Reste tragenden Atomen heterocyclisches mono-, bi- oder tricyclisches Ringsystem mit 3-15 C-Atomen bilden, das gegebenenfalls am N-Atom durch ($C_1$-$C_6$)-Alkyl substituiert sein kann, oder fehlt, falls A = 4-Hydroxyphenyl-($C_1$-$C_4$)-alkanoyl, 4-Hydroxyphenoxyacetyl, 4-Halo-phenyl-($C_1$-$C_4$)-alkanoyl, 4-Halo-phenoxyacetyl oder 4-Halo-benzyloxycarbonyl;

A Tyr, (4-Halo)Phe, 3-Carboxy-1,2,3,4-tetrahydroisochinolin, 3-Carboxy-6-hydroxy-1,2,3,4-tetrahydroisochinolin, 3-Carboxy-7-hydroxy-1,2,3,4-tetrahydroisochinolin, S-Carboxymethylcystein, Glu, Aad, wobei die Aminosäure jeweils als L- oder D-Enantiomer vorliegen kann; 4-Hydroxyphenyl-($C_1$-$C_4$)-alkanoyl; 4-Hydroxyphenoxyacetyl; 4-Halo-phenyl-($C_1$-$C_4$)-alkanoyl; 4-Halo-phenoxyacetyl oder 4-Halo-benzyloxycarbonyl;

B D-Ala, D-Leu, D-Ser, D-Thr, D-Phe oder D-3-(2-Thienyl)alanin;

D Phe, (4-Halo)Phe, (4-($C_1$-$C_4$)-Alkoxy)Phe, Trp oder 3-(2-Thienyl-alanin;

E Thr, Ser oder Gly;

F Asn oder D-Asn;

G Ser, Thr, Ala, Leu, Ile oder Phe;

I Arg, hArg, Lys oder Orn;

K Val, Ile oder Cyclohexyl-alanin;

L Gly, Asp, Glu, Phe, Tyr, Trp oder Gln;

M Ser, Ala, Thr, Leu, Ile, Phe oder Tyr;

P Leu, Phe oder 3-(2-Thienyl)-alanin;

Q Glu, Asp, D-Glu oder D-Asp;

R Nle, Leu, Ile oder O-Methyl-homo-serin;

T eine direkte Bindung, Ser, Gly, Ala, Thr, Ile, Leu, Phe, 3-(2-Thienyl)-alanin oder Gln;

U eine direkte Bindung, Arg oder hArg und

Y $-NH_2$, $-NH-(CH_2)_n-OH$ mit n = 2 - 8 oder

falls U oder T und U für eine direkte Bindung stehen

$-NH-(CH_2)_n-NH_2$, $-NH-(CH_2)_n-NH-C(=NH)NH_2$ mit n = 4 - 10, $\beta$-Ala-$NH_2$ oder $\gamma$-Abu-$NH_2$,

mit der Maßgabe, daß im Falle von Y = $NH_2$ X für einen Rest der Formel II stehen muß, mit Ausnahme der folgenden Peptide der Formel I

Ac-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,

H-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,

bedeuten, sowie deren physiologisch verträgliche Salze.

2. Verbindung der Formel I gemäß Anspruch 1, in welcher

X Wasserstoff; ($C_1$-$C_8$)-Alkyl; ($C_1$-$C_8$)-Alkylcarbonyl; Carboxy-($C_1$-$C_8$)-alkyl oder einen Rest der Formel II;
A 3-Carboxy-1,2,3,4-tetrahydroisochinolin; 3-Carboxy-6-hydroxy-1,2,3,4-tetrahydroisochinolin, 3-Carboxy-7-hydroxy-1,2,3,4-tetrahydroisochinolin, 4-Hydroxyphenyl-($C_1$-$C_4$)-alkanoyl oder 4-Hydroxyphenoxyacetyl;
B D-Ala oder D-Ser;
D Phe oder (4-Halo)Phe;
E Thr oder Ser;
F Asn;
G Ser, Thr oder Ala;
I Arg oder hArg;
K Val oder Ile;
L Gly, Asp, Glu oder Gln;
M Ser, Ala, Thr, Leu oder Ile;
P Leu;
Q Glu oder Asp;
R Nle oder Leu;
T eine direkte Bindung, Ser, Ala, Thr oder Gln;
U eine direkte Bindung, Arg oder hArg und
Y $-NH_2$, oder
falls U oder T und U für eine direkte Bindung stehen
$-NH-(CH_2)_n-NH_2$, $-NH-(CH_2)_n-NH-C(=NH)NH_2$ mit n = 4 - 10,
mit der Maßgabe, daß im Falle von $Y = NH_2$ X für einen Rest der Formel II stehen muß, mit Ausnahme der folgenden Peptide der Formel I
Ac-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,
H-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,

bedeuten, sowie deren physiologisch verträgliche Salze.

3. Verfahren zur Herstellung einer Verbindung der Formel I gemäß einem oder mehreren der Ansprüche 1 und 2, dadurch gekennzeichnet, daß man

a) ein Fragment mit C-terminaler freier Carboxylgruppe oder dessen aktiviertes Derivat mit einem entsprechenden Fragment mit N-terminaler freier Aminogruppe umsetzt

oder

b) das Peptid stufenweise aufbaut,

in der nach (a) oder (b) erhaltenen Verbindung gegebenenfalls eine oder mehrere zum Schutz anderer Funktionen temporär eingeführte Schutzgruppen abspaltet und die so erhaltenen Verbindungen der Formel I gegebenenfalls in ihre physiologisch verträglichen Salze überführt.

4. Verbindung der Formel I gemäß einem oder mehreren der Ansprüche 1 und 2 zur Anwendung als Heilmittel und Diagnostikum.

5. Verbindung der Formel I gemäß einem oder mehreren der Ansprüche 1 und 2 zur Behandlung von Wachstumsstörungen.

6. Verwendung einer Verbindung der Formel I gemäß einem oder mehreren der Ansprüche 1 und 2 als Heilmittel oder Diagnostikum.

7. Pharmazeutische Zubereitung enthaltend seine Verbindung der Formel I gemäß einem oder mehreren der Ansprüche 1 und 2.

8. Verfahren zur Herstellung einer Zubereitung gemäß Anspruch 7, dadurch gekennzeichnet, daß man den Wirkstoff zusammen mit einem physiologisch annehmbaren Träger und gegebenenfalls weiteren Zusatz-, Hilfs- und/oder Konservierungsstoffen in eine geeignete Darreichungsform bringt.

Patentansprüche für folgende Vertragsstaaten: ES, GR

1. Verfahren zur Herstellung einer Verbindung der Formel I

1 2 3 4 5 6 7 8 9 10 11
X - A - B - Asp - Ala - Ile - D - E - F - G - Tyr - Arg -
12 13 14 15 16 17 18 19 20 21 22 23
I - K - Leu - L - Gln - Leu - M - Ala - Arg - I - P - P -
24 25 26 27 28 29
Gln - Q - Ile - R - T - U - Y

in welcher

X Wasserstoff; ($C_1$-$C_8$)-Alkyl; ($C_1$-$C_8$)-Alkylcarbonyl; Carboxy-($C_1$-$C_8$)-alkyl; oder einen Rest der Formel II,

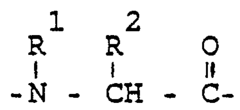

(II)

worin

R' und $R^2$ zusammen mit den diese Reste tragenden Atomen ein heterocyclisches mono-, bi- oder tricyclisches Ringsystem mit 3-15 C-Atomen bilden, das gegebenenfalls am N-Atom durch ($C_1$-$C_6$)-Alkyl substituiert sein kann, oder fehlt, falls A = 4-Hydroxyphenyl-($C_1$-$C_4$)-alkanoyl, 4-Hydroxyphenoxyacetyl, 4-Halo-phenyl-($C_1$-$C_4$)-alkanoyl, 4-Halo-phenoxyacetyl oder 4-Halo-benzyloxycarbonyl;

A Tyr, (4-Halo)Phe, 3-Carboxy-1,2,3,4-tetrahydroisochinolin, 3-Carboxy-6-hydroxy-1,2,3,4-tetrahydroisochinolin, 3-Carboxy-7-hydroxy-1,2,3,4-tetrahydroisochinolin, S-Carboxymethylcystein, Glu, Aad, wobei die Aminosäure jeweils als L- oder D-Enantiomer vorliegen kann; 4-Hydroxyphenyl-($C_1$-$C_4$)-alkanoyl; 4-Hydroxyphenoxyacetyl; 4-Halo-phenyl-($C_1$-$C_4$)-alkanoyl; 4-Halo-phenoxyacetyl oder 4-Halo-benzyloxycarbonyl;

B D-Ala, D-Leu, D-Ser, D-Thr, D-Phe oder D-3-(2-Thienyl)alanin;

D Phe, (4-Halo)Phe, (4-($C_1$-$C_4$)-Alkoxy)Phe, Trp oder 3-(2-Thienyl)-alanin;

E Thr, Ser oder Gly;

F Asn oder D-Asn;

G Ser, Thr, Ala, Leu, Ile oder Phe;

I Arg, hArg, Lys oder Orn;

K Val, Ile oder Cyclohexyl-alanin;

L Gly, Asp, Glu, Phe, Tyr, Trp oder Gln;

M Ser, Ala, Thr, Leu, Ile, Phe oder Tyr;

P Leu, Phe oder 3-(2-Thienyl)-alanin;

Q Glu, Asp, D-Glu oder D-Asp;

R Nle, Leu, Ile oder O-Methyl-homo-serin;

T eine direkte Bindung, Ser, Gly, Ala, Thr, Ile, Leu, Phe, 3-(2-Thienyl)-alanin oder Gln;

U eine direkte Bindung, Arg oder hArg und

Y $-NH_2$, $-NH-(CH_2)_n-OH$ mit n = 2 - 8 oder

falls U oder T und U für eine direkte Bindung stehen

$-NH-(CH_2)_n-NH_2$, $-NH-(CH_2)_n-NH-C(=NH)NH_2$ mit n = 4 - 10, $\beta$-Ala-$NH_2$ oder $\gamma$-Abu-$NH_2$,

mit der Maßgabe, daß im Falle von Y = $NH_2$ X für einen Rest der Formel II stehen muß, mit Ausnahme der folgenden Peptide der Formel I

Ac-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,

H-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,

bedeuten, sowie deren physiologisch verträgliche Salze, dadurch gekennzeichnet, daß man

a) ein Fragment mit C-terminaler freier Carboxylgruppe oder dessen aktiviertes Derivat mit einem entsprechenden Fragment mit N-terminaler freier Aminogruppe umsetzt

oder

b) das Peptid stufenweise aufbaut,

in der nach (a) oder (b) erhaltenen Verbindung gegebenenfalls eine oder mehrere zum Schutz anderer Funktionen temporär eingeführte Schutzgruppen abspaltet und die so erhaltenen Verbindungen der Formel I gegebenenfalls in ihre physiologisch verträglichen Salze überführt.

2. Verfahren gemäß Anspruch 1, dadurch gekennzeichnet, daß man eine Verbindung der Formel I herstellt, in welcher

X Wasserstoff; ($C_1$-$C_8$)-Alkyl; ($C_1$-$C_8$)-Alkylcarbonyl; Carboxy-($C_1$-$C_8$)-alkyl oder einen Rest der Formel II:

A 3-Carboxy-1,2,3,4-tetrahydroisochinolin; 3-Carboxy-6-hydroxy-1,2,3,4-tetrahydroisochinolin, 3-Carboxy-7-hydroxy-1,2,3,4-tetrahydroisochinolin, 4-Hydroxyphenyl-($C_1$-$C_4$)-alkanoyl oder 4-Hydroxyphenoxyacetyl;
B D-Ala oder D-Ser;
D Phe oder (4-Halo)Phe;
E Thr oder Ser;
F Asn;
G Ser, Thr oder Ala;
I Arg oder hArg;
K Val oder Ile;
L Gly, Asp, Glu oder Gln;
M Ser, Ala, Thr, Leu oder Ile;
P Leu;
Q Glu oder Asp;
R Nle oder Leu;
T eine direkte Bindung, Ser, Ala, Thr oder Gln;
U eine direkte Bindung, Arg oder hArg und
Y $-NH_2$, oder
falls U oder T und U für eine direkte Bindung stehen
$-NH-(CH_2)_n-NH_2$, $-NH-(CH_2)_n-NH-C(=NH)NH_2$ mit $n = 4 - 10$,
mit der Maßgabe, daß im Falle von $Y=NH_2$ X für einen Rest der Formel II stehen muß, mit Ausnahme der folgenden Peptide der Formel I
Ac-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-IleNle-Ser-Arg-$NH_2$,
H-Tyr-D-Ala-Asp-Ala-Ile-Phe-Thr-Asn-Ser-Tyr-Arg-Lys-Val-Leu-Glu-Gln-Leu-Ser-Ala-Arg-Lys-Leu-Leu-Gln-Asp-Ile-Nle-Ser-Arg-$NH_2$,

bedeuten, sowie deren physiologisch verträgliche Salze.

3. Verfahren zur Herstellung einer pharmazeutsichen Zubereitung, enthaltend eine Verbindung der Formel I gemäß einem oder mehreren der Ansprüche 1 und 2, dadurch gekennzeichnet, daß man den Wirkstoff zusammen mit einem physiologisch annehmbaren Träger und gegebenenfalls weiteren Zusatz-, Hilfs- und/oder Konservierungsstoffen in eine geeignete Darreichungsform bringt.